# NEW EUROPEAN PATENT SPECIFICATION

(11) **EP 3 049 157 B2**
(45) Date of publication and mention of the opposition decision: **20.04.2022**
(45) Mention of the grant of the patent: 01.08.2018
(21) Application number: 14777990.4
(22) Date of filing: 16.09.2014
(51) Int. Cl.: A61Q 5/12, A61K 8/81, A61K 8/898, A61K 8/89, A61K 8/04

(54) **HAIR COMPOSITIONS COMPRISING LOW VISCOSITY SILICONE POLYMERS**
HAARBEHANDLUNGSMITTEL ENTHALTEND NIEDRIGVISKOSE SILIKONPOLYMERE
COMPOSITIONS CAPILLAIRES COMPRENANT DES POLYMERES SILICONES BASSE VISCOSITE

(30) Priority: 27.09.2013 US 201361883705 P
(43) Date of publication of application: 03.08.2016
(73) Proprietor: The Procter & Gamble Company, Cincinnati, OH 45202 (US)
(72) Inventor: SNYDER, Michael Albert, Cincinnati, Ohio 45202 (US); WEAVER, Martha Jane, Cincinnati, Ohio 45202 (US)
(74) Representative: P&G Patent Germany
(86) International application number: PCT/US2014/055777
(87) International publication number: WO 2015/047785

(56) References cited:
- WO-A1-2013/148904
- WO-A1-2013/149335
- WO-A1-2015/002811
- WO-A1-2015/002812
- WO-A2-2006/138201
- WO-A2-2008/142659
- WO-A2-2013/148778
- US-A1- 2004 048 996
- US-A1- 2011 135 588

## Description

### TECHNICAL FIELD OF THE INVENTION

Provided is a hair conditioning composition comprising silicone polymers. The silicone polymers have lower viscosities, which allow the composition to provide improved conditioning benefits such as smooth feel and reduced friction to both damaged hair and non-damaged hair.

### BACKGROUND OF THE INVENTION

Silicone polymers are strategically important materials in hair conditioning, especially in providing conditioning benefits to hair. Human hair becomes damaged due to, for example, shampooing, combing, permanent waves, and/or coloring the hair. Such damaged hair is often left hydrophilic and/or in a rough condition especially when the hair dries, compared to non-damaged or less damaged hair. Silicone polymers consisting of blocks of silicones and alkylene oxide (e.g., ethylene oxide and propylene oxide groups (EO/PO)) linked with amine- and quat- functional groups have been used to counteract the hydrophilic nature of damaged hair. Silicone blocks are responsible for conditioning and lubrication performance while amine- and quat- functional groups included in the polymer chain further aid deposition during rinsing. In particular, optimum conditioning performance has been observed for silicone blocks of greater than 200 D units. However these materials generally have high viscosities as neat materials. In order to achieve the desired conditioning benefits, these silicone polymers have traditionally been used in blends with silicone copolyols or other diluents or solvents.

Based on the foregoing, there is a need for hair conditioning compositions which provide even greater improved conditioning benefits such as smooth feel and reduced friction on wet hair and dry hair. In addition, there is a need for hair conditioning compositions which provide improved conditioning benefits on damaged hair.

There is also a need for a composition that minimizes the need for additional blend materials in combination with silicone polymers, while delivering the above mentioned combination of benefits with lower cost and complexity than the traditional blend materials.

### SUMMARY OF THE INVENTION

Without being bound by theory, the low viscosity silicone polymers in the hair conditioning compositions of the present invention provide improved conditioning benefits to both damaged hair and non-damaged hair while eliminating the need for a silicone blend. The invention is defined by the appended claims.

In accordance with the invention, the hair conditioning compositions comprise a silicone polymer comprising one or more quaternary groups, at least one silicone block comprising greater than 200 siloxane units, at least one polyalkylene oxide structural unit, and at least one terminal ester group, wherein the silicone polymer has a viscosity from 500 to 50,000 mPa.s, and wherein the a hair conditioning composition comprises a polymeric thickener; and said silicone polymer is defined by the chemical structure Ia or Ib, shown herein below and the polymeric thickener as defined in the claims.

These and additional features provided by the embodiments of the present invention will be more fully understood in view of the following detailed description.

### DETAILED DESCRIPTION OF THE INVENTION

Components of the personal care compositions (e.g., hair conditioning composition) are described below. Also included is a nonexclusive description of various optional and preferred components useful in embodiments of the present invention. While the specification concludes with claims that particularly point out and distinctly claim the invention, it is believed the present invention will be better understood from the following description.

All percentages, parts, and ratios are based upon the total weight of the compositions of the present invention, unless otherwise specified. All such weights as they pertain to listed ingredients are based on the active level and, therefore, do not include solvents or by-products that may be included in commercially available materials, unless otherwise specified. The term "weight percent" may be denoted as "wt.%" herein.

All molecular weights as used herein are weight average molecular weights expressed as grams/mole, unless otherwise specified.

The compositions and methods/processes of the present invention can comprise, consist of, and consist essentially of the essential elements and limitations of the invention described herein, as well as any of the additional or optional ingredients, components, steps, or limitations described herein.

Herein, "mixtures" is meant to include a simple combination of materials and any compounds that may result from their combination.

As used herein, "indicia" means an identifying mark, including text and/or graphics.

As used herein, "image" means a photograph, illustration, and/or other pictorial representation of an object.

Damaged hair is less hydrophobic compared to non-damaged and/or less damaged hair. It is believed that by providing improved hydrophobicity to hair, the hair conditioning composition can provide improved smooth feel and reduced friction to the hair. It is also believed that the improved hydrophobicity to the hair can be provided by some other preferred features of the present invention, for example, the use of additional materials such as silicones, and/or cationic surfactants. Further, without being limited to the theory, it is believed that improved hydrophobicity provides improved tolerance to the hair for humidity in the surrounding circumstances, and thus provides reduced frizziness and/or fly-aways on rainy and/or humid days.

The hair conditioning composition of the present invention has a pH of alternatively from about 2 to about 9, or alternatively from about 3 to about 7.

### A. Silicone Polymer Containing Quaternary Groups

The compositions of the present invention comprise a low viscosity silicone polymer.

Without being bound by theory, this low viscosity silicone polymer provides improved conditioning benefits such as smooth feel, reduced friction, and prevention of hair damage, while eliminating the need for a silicone blend.

Structurally, the silicone polymer is a polyorganosiloxane compound comprising one or more quaternary ammonium groups, at least one silicone block comprising greater than 200 siloxane units, at least one polyalkylene oxide structural unit, and at least one terminal ester group. In one or more embodiments, the silicone block may comprise between 300 to 500 siloxane units.

The silicone polymer may be present in an amount of from about 0.05% to about 15%, alternatively from about 0.1% to about 10%, alternatively from about 0.15% to about 5%, and alternatively from about 0.2% to about 4% by weight of the composition.

The polyorganosiloxane compounds according to the invention have the general formulas (Ia) and (Ib):

M-Y-[-(N⁺R₂-T-N⁺R₂)-Y-I]ₘ-[-(NR²-A-E-A'-NR²)-Y-]ₖ-M (Ia)

M-Y-[-(N⁺R₂-T-N⁺R₂)-Y-]ₘ-[-(N⁺R²⁻A-E-A'-N⁺R²₂)-Y-]ₖ-M (Ib)

wherein:
m is > 0, preferred 0.01 to 100, more preferred 0.1 to 100, even more preferred 1 to 100, specifically 1 to 50, more specifically 1 to 20, even more specifically 1 to 10,
k is an average value of from >0 to 50, or alternatively from 1 to 20, or alternatively from 1 to 10,
M represents a terminal group, comprising terminal ester groups selected from

   -OC(O)-Z

   -OS(O)₂-Z

   -OS(O₂)O-Z

   -OP(O)(O-Z)OH

   -OP(O)(O-Z)₂

   wherein Z is selected from monovalent organic residues having up to 40 carbon atoms, optionally comprising one or more hetero atoms.

A and A' each are independently from each other selected from a single bond or a divalent organic group having up to 10 carbon atoms and one or more hetero atoms, and

E is a polyalkylene oxide group of the general formula:

-[CH₂CH₂O]_{q}[CH₂CH(CH₃)O]ᵣ-CH₂CH(C₂H₅)O]ₛ-

wherein q=0 to 200, r=0 to 200, s=0 to 200, and q+r+s = 1 to 600.

R² is selected from hydrogen or R,

R is selected from monovalent organic groups having up to 22 carbon atoms and optionally one or more heteroatoms, and wherein the free valencies at the nitrogen atoms are bound to carbon atoms,

Y is a group of the formula:

   -K-S-K- and -A-E-A'-or-A'-E-A-,

   with S= 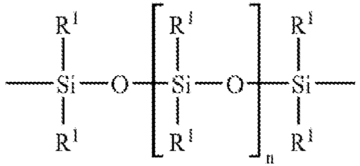 wherein R1 = C₁-C₂₂-alkyl, C₁-C₂₂-fluoralkyl or aryl; n=200 to 1000, and these can be identical or different if several S Groups are present in the polyorganosiloxane compound.

K is a bivalent or trivalent straight chain, cyclic and/or branched C₂-C₄₀ hydrocarbon residue which is optionally interrupted by-O-,-NH-, trivalent N, -NR¹-,-C(O)-, -C(S)-, and optionally substituted with-OH, wherein R¹ is defined as above,

T is selected from a divalent organic group having up to 20 carbon atoms and one or more hetero atoms.

The residues K may be identical or different from each other. In the -K-S-K- moiety, the residue K is bound to the silicon atom of the residue S via a C-Si-bond.

Due to the possible presence of amine groups (-(NR²-A-E-A'-NR²)-) in the polyorganosiloxane compounds, they may have protonated ammonium groups, resulting from the protonation of such amine groups with organic or inorganic acids. Such compounds are sometimes referred to as acid addition salts of the polyorganosiloxane compounds according to the invention.

In an embodiment the molar ratio of the quaternary ammonium groups b) and the terminal ester groups c) is less than 100 : 20, alternatively less than 100 : 30, and alternatively less than 100 : 50. The ratio can be determined by ¹³C-NMR.

In a further embodiment, the polyorganosiloxane composition may comprise:
A) at least one polyorganosiloxane compound, comprising a) at least one polyorganosiloxane group, b) at least one quaternary ammonium group, c) at least one terminal ester group, and d) at least one polyalkylene oxide group (as defined before),
B) at least one polyorganosiloxane compound, comprising at least one terminal ester group, different from compound A).

In the definition of component A) it can be referred to the description of the polyorganosiloxane compounds of the invention. The polyorganosiloxane compound B) differs from the polyorganosiloxane compound A) alternatively in that it does not comprise quaternary ammonium groups. Preferred polyorganosiloxane compounds B) result from the reaction of monofunctional organic acids, in particular carboxylic acids, and polyorganosiloxane containing bisepoxides.

In the polyorganosiloxane compositions according to the invention the weight ratio of compound A) to compound B) is alternatively less than 90 : 10. Or in other words, the content of component B) is at least 10 weight percent. In a further preferred embodiment of the polyorganosiloxane compositions according to the invention in compound A) the molar ratio of the quaternary ammonium groups b) and the terminal ester groups c) is less than 100 : 10, even more preferred is less than 100 : 15 and is most preferred less than 100 : 20.

The silicone polymer has a viscosity at 20°C and a shear rate of 0.1s⁻¹ (plate-plate system, plate diameter 40mm, gap width 0.5mm) of from 500 to 50,000 mPa.s, or alternatively from 500 to 20,000 mPa.s. In further embodiments, the viscosities of the neat polymers may range from 500 to 10,000 mPa.s, or alternatively 500 to 5000 mPa.s determined at 20 °C and a shear rate of 0.1 s⁻¹.

In addition to the above listed silicone polymers, the following preferred compositions are provided below. For example, in the polyalkylene oxide group E of the general formula:

-[CH₂CH₂O]_{q}-[CH₂CH(CH₃)O]ᵣ-[CH₂CH(C₂H₅)O]ₛ-

wherein the q, r, and s indices may be defined as follows:
q=0 to 200, or alternatively from 0 to 100, or alternatively from 0 to 50, or alternatively from 0 to 20,
r=0 to 200, or alternatively from 0 to 100, or alternatively from 0 to 50, or alternatively from 0 to 20,
s=0 to 200, or alternatively from 0 to 100, or alternatively from 0 to 50, or alternatively from 0 to 20,
and q+r+s = 1 to 600, or alternatively from 1 to 100, or alternatively from 1 to 50, or alternatively from 1 to 40.

For polyorganosiloxane structural units with the general formula S:

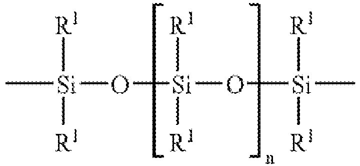

R¹=C₁-C₂₂-alkyl, C₁-C₂₂-fluoralkyl or aryl; n= from 200 to 1000, or alternatively from 300 to 500, K (in the group -K-S-K-) is alternatively a bivalent or trivalent straight chain, cyclical or branched C₂-C₂₀ hydrocarbon residue which is optionally interrupted by-O-,-NH-, trivalent N,-NR¹-,-C(O)-,-C(S)-, and optionally substituted with-OH.

In specific embodiments, R¹ is C₁-C₁₈ alkyl, C₁-C₁₈ fluoroalkyl and aryl. Furthermore, R¹ is alternatively C₁-C₁₈ alkyl, C₁-C₆ fluoroalkyl and aryl. Furthermore, R¹ is alternatively C₁-C₆ alkyl, C₁-C₆ fluoroalkyl, alternatively C₁-C₄ fluoroalkyl, and phenyl. Most alternatively, R¹ is methyl, ethyl, trifluoropropyl and phenyl.

As used herein, the term "C₁-C₂₂ alkyl" means that the aliphatic hydrocarbon groups possess from 1 to 22 carbon atoms which can be straight chain or branched. Methyl, ethyl, propyl, n-butyl, pentyl, hexyl, heptyl, nonyl, decyl, undecyl, isopropyl, neopentyl and 1,2,3-trimethyl hexyl moieties serve as examples.

Further as used herein, the term "C₁-C₂₂ fluoroalkyl" means aliphatic hydrocarbon compounds with 1 to 22 carbon atoms which can be straight chain or branched and are substituted with at least one fluorine atom. Monofluormethyl, monofluoroethyl, 1,1,1-trifluorethyl, perfluoroethyl, 1,1,1-trifluoropropyl, 1,2,2-trifluorobutyl are suitable examples.

Moreover, the term "aryl" means unsubstituted or phenyl substituted once or several times with OH, F, Cl, CF₃, C₁-C₆ alkyl, C₁-C₆ alkoxy, C₃-C₇ cycloalkyl, C₂-C₆ alkenyl or phenyl. Aryl may also mean naphthyl.

For the embodiments of the polyorganosiloxanes, the positive charges resulting from the ammonium group(s), are neutralized with inorganic anions such as chloride, bromide, hydrogen sulfate, sulfate, or organic anions, like carboxylates deriving from C₁-C₃₀ carboxylic acids, for example acetate, propionate, octanoate, especially from C₁₀-C₁₈ carboxylic acids, for example decanoate, dodecanoate, tetradecanoate, hexadecanoate, octadecanoate and oleate, alkylpolyethercarboxylate, alkylsulphonate, arylsulphonate, alkylarylsulphonate, alkylsulphate, alkylpolyethersulphate, phosphates derived from phosphoric acid mono alkyl/aryl ester and phosphoric acid dialkyl/aryl ester. The properties of the polyorganosiloxane compounds can be, *inter alia,* modified based upon the selection of acids used.

The quaternary ammonium groups are usually generated by reacting the di-tertiary amines with an alkylating agents, selected from in particular di-epoxides (sometimes referred to also as bisepoxides) in the presence of mono carboxylic acids and difunctional dihalogen alkyl compounds.

Also disclosed herein are polyorganosiloxane compounds of the general formulas (Ia) and (Ib):

M-Y-[-(N⁺R₂-T-N⁺R₂)-Y-]ₘ-[-(NR²-A-E-A'-NR²)-Y-]ₖ-M (Ia)

M-Y-[-(N⁺R₂-T-N⁺R₂)-Y-]ₘ-[-(N⁺R²₂-A-E-A'-N⁺R²₂)-Y-]ₖ-M (Ib)

wherein each group is as defined above; however, the repeating units are in a statistical arrangement (i.e., not a block-wise arrangement).

In a further embodiment, the molar ratio of the polyorganosiloxane-containing repeating group-K-S-K-and the polyalkylene repeating group -A-E-A'- or -A'-E-A- is between 100:1 and 1:100, or alternatively between 20:1 and 1:20, or alternatively between 10:1 and 1:10.

In the group -(N⁺R₂-T-N⁺R₂)-, R may represent a monovalent straight chain, cyclic or branched C₁-C₂₀ hydrocarbon radical, which can be interrupted by one or more -O- , -C(O)- and can be substituted by-OH, T may represent a divalent straight-chain, cyclic, or branched C₁-C₂₀ hydrocarbon radical, which can be interrupted by -O- , -C(O)- and can be substituted by hydroxyl.

Various monofunctional organic acids may be utilized to yield the esters. Exemplary embodiments include C₁-C₃₀ carboxylic acids, for example C₂, C₃, C₈ acids, C₁₀-C₁₈ carboxylic acids, for example C₁₂, C₁₄, C₁₆ acids, saturated, unsaturated and hydroxyl functionalized C₁₈ acids, alkylpolyethercarboxylic acids, alkylsulphonic acids, ar-ylsulphonic acids, alkylarylsulphonic acids, alkylsulphuric acids, alkylpolyethersulphuric acids, phosphoric acid mono alkyl/aryl esters and phosphoric acid dialkyl/aryl esters.

Further performance improvements may optionally be achieved by pre-dispersing the silicone polymer in a small particle emulsion (less than 1 micron) prior to adding it to the conditioner base.

The term "emulsion" in this patent application describes any stable emulsion or dispersion of the silicone polymer, separately prepared and used as one of the components of a conditioner composition.

Stable means that the viscosity, particle size, and other important characteristics of the emulsion do not significantly change over reasonable time under exposure to typical temperature, moisture, pressure, shear, light and other environmental conditions that the pre-emulsion is exposed during packing, storage, and transportation

Making the small particle emulsion may require pre-emulsifying the silicone polymer before their addition to the conditioning composition. A non-limiting example of a method of making is provided below. All oil soluble components are mixed in a vessel. Heat may be applied to allow mixture to liquidify. All water soluble components are mixed in a separate vessel and heated to about same temperature as the oil phase. The oil phase and aqueous phase are mixed under a high shear mixer (example, Turrax mixer by IKA). The particle size of the silicone polymer is in the range of about 0.01 µm to about 5 µm, alternatively from 0.05 µm to about 1 µm, alternatively from about 0.1 µm to about 0.5 µm. High energy mixing device may be used to achieve desired particle size. High energy mixing device include, but not limited to Microfluidizer from Microfluidics Corp., Sonolator from Sonic Corp., Colloid mill from Sonic Corp.

The emulsifiers which may be selected for each the silicone may be guided by the Hydrophilic-Lipophilic-Balance value (HLB value) of emulsifiers. Suitable range of HLB value may be 6-16, alternatively 8-14. Emulsifiers with a HLB higher than 10 are water soluble. Emulsifiers with low HLB are lipid soluble. To obtain suitable HLB value, a mixture of two or more emulsifiers may be used. Suitable emulsifiers include non-ionic, cationic, anionic and amphoteric emulsifiers.

The concentration of the emulsifier in the emulsion and the emulsifications of the silicone polymer should be sufficient to achieve desired particle size and emulsion stability, and generally may range from about 0.1 wt%-about 50 wt%, from about 1 wt%-about 30 wt%, from about 2 wt%-about 20 wt%, for example.

The optional use of a pre-emulsified dispersion of the silicone may present multiple advantages including: (i) The small particle size of the silicones in the emulsion leads to more even deposition and reduces island-like spotty deposits; and (ii) the more even deposition is more favorable for providing smoothness for hair/skin surfaces, easier combing, and enhanced hair volume.

### B. Polymeric Thickener

The hair conditioning composition includes rheology modifiers to adjust the rheological characteristics of the composition for better feel, in-use properties and the suspending stability of the composition. For example, the rheological properties may be adjusted so that the composition remains uniform during its storage and transportation and does not drip undesirably onto other areas of the body, clothing or home furnishings during its use. Any suitable rheology modifier may be used. In an embodiment, the hair conditioning composition may comprise from about 0.01% to about 3% of a rheology modifier, alternatively from about 0.1% to about 1% of a rheology modifier.

The rheology modifiers include acrylamide/ammonium acrylate copolymer (and) polyisobutene (and) polysorbate 20; acrylamide/sodium acryloyldimethyl taurate copolymer (and) isohexadecane (and) polysorbate 80; acrylates copolymer; acrylates/beheneth-25 methacrylate copolymer; acrylates/C10-C30 alkyl acrylate crosspolymer; acrylates/steareth-20 itaconate copolymer; ammonium polyacrylate (and) Isohexadecane (and) PEG-40 castor oil; C12-16 alkyl PEG-2 hydroxypropylhydroxyethyl ethylcellulose (HM-EHEC); carbomer; crosslinked polyvinylpyrrolidone (PVP); dibenzylidene sorbitol; hydroxyethyl ethylcellulose (EHEC); hydroxypropyl methylcellulose (HPMC); hydroxypropyl methylcellulose (HPMC); hydroxypropylcellulose (HPC); methylcellulose (MC); methylhydroxyethyl cellulose (MEHEC); PEG-150/decyl alcohol/SMDI copolymer; PEG-150/stearyl alcohol/SMDI copolymer; polyacrylamide (and) C13-14 isoparaffin (and) laureth-7; polyacrylate 13 (and) polyisobutene (and) polysorbate 20; polyacrylate crosspolymer-6; polyamide-3; polyquaternium-37 (and) hydrogenated polydecene (and) trideceth-6; polyurethane-39; sodium acrylate/acryloyldimethyltaurate/dimethylacrylamide crosspolymer (and) isohexadecane (and) polysorbate 60; or sodium polyacrylate. Exemplary commercially-available rheology modifiers include ACULYN^{™} 28, Klucel M CS, Klucel H CS, Klucel G CS, SYLVACLEAR AF1900V, SYLVACLEAR PA1200V, Benecel E10M, Benecel K35M, Optasense RMC70, ACULYN^{™}33, ACULYN^{™}46, ACULYN^{™}22, ACULYN^{™}44, Carbopol Ultrez 20, Carbopol Ultrez 21, Carbopol Ultrez 10, Carbopol 1342, Sepigel^{™} 305, Simulgel^{™}600, Sepimax Zen, and/or combinations thereof.

### C. Carrier

Hair conditioning compositions typically comprise a carrier, which may be present at a level of from about 20 wt% to about 99 wt% , and/or from about 60 wt% to about 85 wt%. The carrier may comprise water, organic solvents (miscible or non-miscible with water), silicone solvents and/or mixtures thereof. The solvents should be dermatologically acceptable. The carrier may not comprise more than about 2, about 1, about 0.5, about 0.2, about 0.1, and/or about 0.05 wt% of non-volatile solvent. Significantly higher concentration of non-volatile carrier will increase hair weigh-down.and greasy hair feel. In one emobodiment the carrier may comprise water with minimal or no significant concentrations of organic solvent, except as otherwise incidentally incorporated into the composition as minor ingredients of other components. Water, organic and silicone solvents that have boiling points below or equal to 250°C are volatile solvents. Solvents with boiling points above 250°C are considered non-volatile.

The carrier useful in embodiments of the hair conditioning composition includes water and water solutions of lower alkyl alcohols and polyhydric alcohols. The lower alkyl alcohols useful herein are monohydric alcohols having 1 to 6 carbons, in one aspect, ethanol and isopropanol. Exemplary polyhydric alcohols useful herein include glycols, glycerine and other diols.

### D. Additional Components

The composition of the present invention may include other additional components, which may be selected by the artisan according to the desired characteristics of the final product and which are suitable for rendering the composition more cosmetically or aesthetically acceptable or to provide them with additional usage benefits. Such other additional components generally are used individually at levels of from about 0.001% to about 10%, alternatively up to about 5% by weight of the composition.

A wide variety of other additional components can be formulated into the present compositions. These include: other conditioning agents such as hydrolysed collagen with tradename Peptein 2000 available from Hormel, water soluble and water insoluble vitamins such as vitamin A, D, B₁, B₂, B₆, B₁₂, C, biotin, vitamin E with tradename Emix-d available from Eisai, panthenol available from Roche, pantothenic acid, panthenyl ethyl ether available from Roche, and their derivatives; hydrolysed keratin, proteins, plant extracts, and nutrients; emollients such as PPG-3 myristyl ether with tradename Varonic APM available from Goldschmidt, Trimethyl pentanol hydroxyethyl ether, PPG-11 stearyl ether with tradename Varonic APS available from Goldschmidt, Stearyl heptanoate with tradename Tegosoft SH available from Goldschmidt, Lactil (mixture of Sodium lactate, Sodium PCA, Glycine, Fructose, Urea, Niacinamide, Glucosamine, Inositol, Sodium Benzoate, and Lactic acid) available from Goldschmidt, Sodium lactate, Sodium PCA, Glycine, Fructose, Urea, Niacinamide, Glucosamine, Inositol, Sodium Benzoate, Lactic acid, Ethyl hexyl palmitate with tradename Saracos available from Nishin Seiyu and with tradename Tegosoft OP available from Goldschmidt; hair-fixative polymers such as amphoteric fixative polymers, cationic fixative polymers, anionic fixative polymers, nonionic fixative polymers, and silicone grafted copolymers; preservatives such as benzyl alcohol, methyl paraben, propyl paraben and imidazolidinyl urea; pH adjusting agents, such as citric acid, sodium citrate, succinic acid, phosphoric acid, sodium hydroxide, sodium carbonate; salts, in general, such as potassium acetate and sodium chloride; coloring agents, such as any of the FD&C or D&C dyes, oxidative dyes and interference pigments; hair oxidizing (bleaching) agents, such as hydrogen peroxide, perborate and persulfate salts, carbonate; hair reducing agents such as the thioglycolates; perfumes; and sequestering agents, such as disodium ethylenediamine tetra-acetate; ultraviolet and infrared screening and absorbing agents such as octyl salicylate; antimicrobial agents; suspending agents; viscosity modifiers; nonvolatile solvents or diluents (water soluble and insoluble), pearlescent aids, foam boosters, additional surfactants or nonionic cosurfactants, pediculocides, chelants, skin active agents, sunscreens, UV absorbers, and, water soluble and insoluble amino acids such as asparagine, alanin, indole, glutamic acid, tyrosine, tryptamine, and their salts; and antidandruff agents such as zinc pyrithione, pyridinethione salts, azoles, climbazole, octopirox, salicylic acid, selenium sulfide, particulate sulfur, mixtures thereof.

### 1. Silicone

The composition of the present invention may further comprise a silicone compound, in addition to the silicone polymer containing quaternary groups. The silicone compound can be included in an amount of from about 0.1% to about 10%, alternatively from about 0.25% to about 8%, still alternatively from about 0.5% to about 3% by weight of the composition.

The silicone compounds hereof can include volatile soluble or insoluble, or nonvolatile soluble or insoluble silicone conditioning agents. By soluble what is meant is that the silicone compound is miscible with the carrier of the composition so as to form part of the same phase. By insoluble what is meant is that the silicone forms a separate, discontinuous phase from the carrier, such as in the form of an emulsion or a suspension of droplets of the silicone. The silicone compounds herein may be made by conventional polymerization, or emulsion polymerization.

The silicone compounds for use herein will alternatively have a viscosity of from about 1,000 to about 2,000,000 centistokes at 25°C, alternatively from about 10,000 to about 1,800,000 centistokes, and alternatively from about 25,000 to about 1,500,000 centistokes. The viscosity can be measured by means of a glass capillary viscometer as set forth in Dow Corning Corporate Test Method CTM0004, July 20, 1970. Silicone compounds of high molecular weight may be made by emulsion polymerization.

Silicone compounds useful herein include polyalkyl polyaryl siloxanes, polyalkyleneoxide-modified siloxanes, silicone resins, amino-substituted siloxanes, and mixtures thereof. The silicone compound is alternatively selected from the group consisting of polyalkyl polyaryl siloxanes, polyalkyleneoxide-modified siloxanes, silicone resins, and mixtures thereof, and alternatively from one or more polyalkyl polyaryl siloxanes.

Polyalkyl polyaryl siloxanes useful here in include those with the following structure (XIV) 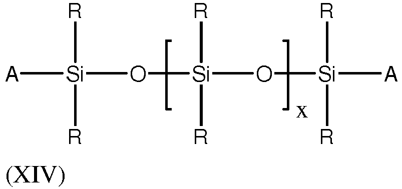 wherein R is alkyl or aryl, and x is an integer from about 7 to about 8,000. A represents groups which block the ends of the silicone chains. The alkyl or aryl groups substituted on the siloxane chain (R) or at the ends of the siloxane chains (A) can have any structure as long as the resulting silicone remains fluid at room temperature, is dispersible, is neither irritating, toxic nor otherwise harmful when applied to the hair, is compatible with the other components of the composition, is chemically stable under normal use and storage conditions, and is capable of being deposited on and conditions the hair. Suitable A groups include hydroxy, methyl, methoxy, ethoxy, propoxy, and aryloxy. The two R groups on the silicon atom may represent the same group or different groups. Alternatively, the two R groups represent the same group. Suitable R groups include methyl, ethyl, propyl, phenyl, methylphenyl and phenylmethyl. The preferred silicone compounds are polydimethylsiloxane, polydiethylsiloxane, and polymethylphenylsiloxane. Polydimethylsiloxane, which is also known as dimethicone, is especially preferred. The polyalkylsiloxanes that can be used include, for example, polydimethylsiloxanes. These silicone compounds are available, for example, from Momentive Performance Materials in their Element 14^{®} series, and from Dow Corning in their Dow Corning 200 series. Polymethylphenylsiloxanes, for example, from Momentive Performance Materials as SF 1550 methyl phenyl fluid or from Dow Corning as 556 Cosmetic Grade Fluid, are useful herein.

Also preferred, for enhancing the shine characteristics of hair, are highly arylated silicone compounds, such as highly phenylated polyethyl silicone having refractive index of about 1.46 or higher, especially about 1.52 or higher. When these high refractive index silicone compounds are used, they should be mixed with a spreading agent, such as a surfactant or a silicone resin, as described below to decrease the surface tension and enhance the film forming ability of the material.

Another polyalkyl polyaryl siloxane that can be especially useful is a silicone gum. The term "silicone gum," as used herein, means a polyorganosiloxane material having a viscosity at 25°C of greater than or equal to 1,000,000 centistokes. It is recognized that the silicone gums described herein can also have some overlap with the above-disclosed silicone compounds. This overlap is not intended as a limitation on any of these materials. Silicone gums are described by Petrarch, and others including U.S. Patent No. 4,152,416, to Spitzer et al., issued May 1, 1979 and Noll, Walter, Chemistry and Technology of Silicones, New York: Academic Press 1968. Also describing silicone gums are Momentive Performance Materials Silicone Rubber Product Data Sheets SE 30, SE 33, SE 54 and SE 76.

The "silicone gums" will typically have a mass molecular weight in excess of about 200,000, generally between about 200,000 and about 1,000,000. Specific examples include polydimethylsiloxane, poly(dimethylsiloxane methylvinylsiloxane) copolymer, poly(dimethylsiloxane diphenylsiloxane methylvinylsiloxane) copolymer and mixtures thereof.

Polyalkyleneoxide-modified siloxanes useful herein include, for example, polypropylene oxide modified and polyethylene oxide modified polydimethylsiloxane. These materials are also known as dimethicone copolyols.

Silicone resins, which are highly crosslinked polymeric siloxane systems, are useful herein. The crosslinking is introduced through the incorporation of tri-functional and tetra-functional silanes with mono-functional or di-functional, or both, silanes during manufacture of the silicone resin. As is well understood in the art, the degree of crosslinking that is required in order to result in a silicone resin will vary according to the specific silane units incorporated into the silicone resin. In general, silicone materials which have a sufficient level of trifunctional and tetrafunctional siloxane monomer units, and hence, a sufficient level of crosslinking, such that they dry down to a rigid, or hard, film are considered to be silicone resins. The ratio of oxygen atoms to silicon atoms is indicative of the level of crosslinking in a particular silicone material. Silicone materials which have at least about 1.1 oxygen atoms per silicon atom will generally be silicone resins herein. Alternatively, the ratio of oxygen:silicon atoms is at least about 1.2:1.0. Silanes used in the manufacture of silicone resins include monomethyl-, dimethyl-, trimethyl-, monophenyl-, diphenyl-, methylphenyl-, monovinyl-, and methylvinylchlorosilanes, and tetrachlorosilane, with the methyl substituted silanes being most commonly utilized. Preferred resins are offered by Momentive Performance Materials as SS4230 and SS4267. Commercially available silicone resins will generally be supplied in a dissolved form in a low viscosity volatile or nonvolatile silicone fluid. The silicone resins for use herein should be supplied and incorporated into the present compositions in such dissolved form, as will be readily apparent to those skilled in the art. Without being bound by theory, it is believed that the silicone resins can enhance deposition of other silicone compounds on the hair and can enhance the glossiness of hair with high refractive index volumes.

Other useful silicone resins are silicone resin powders such as the material given the CTFA designation polymethylsilsequioxane, which is commercially available as Tospearl^{™} from Momentive Performance Materials..

Silicone resins can conveniently be identified according to a shorthand nomenclature system well known to those skilled in the art as the "MDTQ" nomenclature. Under this system, the silicone is described according to the presence of various siloxane monomer units which make up the silicone. Briefly, the symbol M denotes the mono-functional unit (CH₃)₃SiO_{0.5}; D denotes the difunctional unit (CH₃)₂SiO; T denotes the trifunctional unit (CH₃)SiO_{1.5}; and Q denotes the quadri- or tetra-functional unit SiO₂. Primes of the unit symbols, e.g., M', D', T', and Q' denote substituents other than methyl, and must be specifically defined for each occurrence. Typical alternate substituents include groups such as vinyl, phenyl, amino, hydroxyl, etc. The molar ratios of the various units, either in terms of subscripts to the symbols indicating the total number of each type of unit in the silicone, or an average thereof, or as specifically indicated ratios in combination with molecular weight, complete the description of the silicone material under the MDTQ system. Higher relative molar amounts of T, Q, T' and/or Q' to D, D', M and/or or M' in a silicone resin is indicative of higher levels of crosslinking. As discussed before, however, the overall level of crosslinking can also be indicated by the oxygen to silicon ratio.

The silicone resins for use herein which are preferred are MQ, MT, MTQ, MQ and MDTQ resins. Thus, the preferred silicone substituent is methyl. Especially preferred are MQ resins wherein the M:Q ratio is from about 0.5:1.0 to about 1.5:1.0 and the average molecular weight of the resin is from about 1000 to about 10,000.

Amino-substituted siloxanes useful herein include those represented by the following structure (XV) 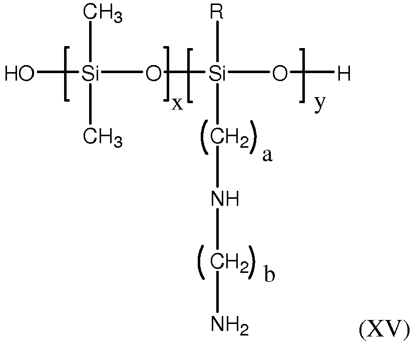 wherein R is CH₃ or OH, x and y are integers which depend on the molecular weight, the average molecular weight alternatively being approximately between 5,000 and 10,000; both a and b denote an integer from 2 to 8. This polymer is also known as "amodimethicone".

Suitable amino-substituted siloxane fluids include those represented by the formula (XVI)

(R₁)ₐG₃₋ₐ-Si-(-OSiG₂)ₙ-(-OSiG_{b}(R₁)_{2-b)m}-O-SiG₃₋ₐ(R₁)ₐ (XVI)

in which G is chosen from the group consisting of hydrogen, phenyl, OH, C₁-C₈ alkyl and alternatively methyl; a is 0 or an integer having a value from 1 to 3, alternatively 1; b is 0, 1 or 2, alternatively 1; n is a number from 0 to 1,999; m is an integer from 0 to 1,999; the sum of n and m is a number from 1 to 2,000; a and m are not both 0; R₁ is a monovalent radical of formula CqH_{2q}L in which q is an integer from 2 to 8 and L is chosen from the groups

-N(R₂)CH₂-CH₂-N(R₂)₂;

-N(R₂)₂;

-N(R₂)⁺₃A⁻;

and

-N(R₂)CH₂-CH₂-NR₂H⁺A⁻

in which R² is chosen from the group consisting of hydrogen, phenyl, benzyl, a saturated hydrocarbon radical, alternatively an alkyl radical containing from 1 to 20 carbon atoms, and A denotes a halide ion.

Highly preferred amino silicones are those corresponding to formula (XVI) wherein m=0, a=1, q=3, G=methyl, n is alternatively from about 1500 to about 1700, alternatively about 1600; and L is -N(CH₃)₂ or -NH₂, alternatively -NH₂. Another highly preferred amino silicones are those corresponding to formula (XVI) wherein m=0, a=1, q=3, G=methyl, n is alternatively from about 400 to about 600, alternatively about 500; and L is -N(CH₃)₂ or -NH₂, alternatively -NH₂. Such highly preferred amino silicones can be called as terminal aminosilicones, as one or both ends of the silicone chain are terminated by nitrogen containing group.

An especially preferred amino-substituted siloxane corresponding to formula (XVI) is the polymer known as "trimethylsilylamodimethicone," of formula (XVII):

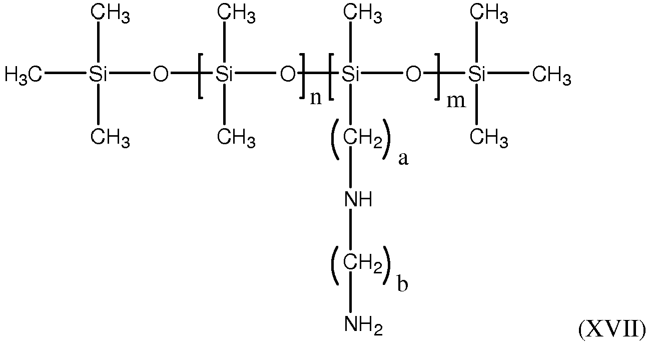

In this formula n and m are selected depending on the molecular weight of the compound desired; both a and b denote an integer from 2 to 8.

In one embodiment of the present invention, the silicone compound may be contained in the composition in the form of a silicone emulsion. The silicone emulsion herein may be a predispersed stable emulsion comprising at least a surfactant, a silicone compound, and water. The surfactant useful herein is any known to the artisan. Silicone emulsions with a high internal phase viscosity are preferred.

In alternative embodiments of the present invention, the above-noted silicone-based quaternary ammonium compounds may be combined with the silicone polymers described in section A (entitled Silicone Polymer Containing Quaternary Groups) of the instant specification.

### 2. Polysorbate

The hair conditioning composition of the present invention may contain a polysorbate, in view of adjusting rheology. Preferred polysorbate useful herein includes, for example, polysorbate-20, polysorbate-21, polysorbate-40, polysorbate-60, and mixtures thereof. Highly preferred is polysorbate-20.

The polysorbate can be contained in the composition at a level by weight of alternatively from about 0.01% to about 5%, alternatively from about 0.05% to about 2%.

### 3. Polypropylene Glycol

Polypropylene glycol useful herein are those having a weight average molecular weight of from about 200 g/mol to about 100,000 g/mol, alternatively from about 1,000 g/mol to about 60,000 g/mol. Without intending to be limited by theory, it is believed that the polypropylene glycol herein deposits onto, or is absorbed into hair to act as a moisturizer buffer, and/or provides one or more other desirable hair conditioning benefits.

The polypropylene glycol useful herein may be either water-soluble, water-insoluble, or may have a limited solubility in water, depending upon the degree of polymerization and whether other moieties are attached thereto. The desired solubility of the polypropylene glycol in water will depend in large part upon the form (e.g., leave-on, or rinse-off form) of the hair conditioning composition. For example, a rinse-off hair conditioning composition, it is preferred that the polypropylene glycol herein has a solubility in water at about 25°C of less than about 1 g/100 g water, alternatively a solubility in water of less than about 0.5 g/100 g water, and alternatively a solubility in water of less than about 0.1 g/100 g water.

The polypropylene glycol can be included in the hair conditioning composition of the present invention at a level of, alternatively from about 0.01% to about 10%, alternatively from about 0.05% to about 6%, still alternatively from about 0.1% to about 3% by weight of the composition.

### 4. Low Melting Point Oil

Low melting point oils useful herein are those having a melting point of less than about 25°C. The low melting point oil useful herein is selected from the group consisting of: hydrocarbon having from about 10 to about 40 carbon atoms; unsaturated fatty alcohols having from about 10 to about 30 carbon atoms such as oleyl alcohol; unsaturated fatty acids having from about 10 to about 30 carbon atoms; fatty acid derivatives; fatty alcohol derivatives; ester oils such as pentaerythritol ester oils, trimethylol ester oils, citrate ester oils, and glyceryl ester oils; poly α-olefin oils; and mixtures thereof. Preferred low melting point oils herein are selected from the group consisting of: ester oils such as pentaerythritol ester oils, trimethylol ester oils, citrate ester oils, and glyceryl ester oils; poly α-olefin oils; and mixtures thereof,

Particularly useful pentaerythritol ester oils and trimethylol ester oils herein include pentaerythritol tetraisostearate, pentaerythritol tetraoleate, trimethylolpropane triisostearate, trimethylolpropane trioleate, and mixtures thereof. Such compounds are available from Kokyo Alcohol with tradenames KAKPTI, KAKTTI, and Shin-nihon Rika with tradenames PTO, ENUJERUBU TP3SO.

Particularly useful citrate ester oils herein include triisocetyl citrate with tradename CITMOL 316 available from Bernel, triisostearyl citrate with tradename PELEMOL TISC available from Phoenix, and trioctyldodecyl citrate with tradename CITMOL 320 available from Bernel.

Particularly useful glyceryl ester oils herein include triisostearin with tradename SUN ESPOL G-318 available from Taiyo Kagaku, triolein with tradename CITHROL GTO available from Croda Surfactants Ltd., trilinolein with tradename EFADERMA-F available from Vevy, or tradename EFA-GLYCERIDES from Brooks.

Particularly useful poly α-olefin oils herein include polydecenes with tradenames PURESYN 6 having a number average molecular weight of about 500 and PURESYN 100 having a number average molecular weight of about 3000 and PURESYN 300 having a number average molecular weight of about 6000 available from Exxon Mobil Co.

### 5. Cationic Polymer

Cationic polymers useful herein are those having a weight average molecular weight of at least about 5,000, typically from about 10,000 to about 10 million, alternatively from about 100,000 to about 2 million.

Suitable cationic polymers include, for example, copolymers of vinyl monomers having cationic amine or quaternary ammonium functionalities with water soluble spacer monomers such as acrylamide, methacrylamide, alkyl and dialkyl acrylamides, alkyl and dialkyl methacrylamides, alkyl acrylate, alkyl methacrylate, vinyl caprolactone, and vinyl pyrrolidone. Other suitable spacer monomers include vinyl esters, vinyl alcohol (made by hydrolysis of polyvinyl acetate), maleic anhydride, propylene glycol, and ethylene glycol. Other suitable cationic polymers useful herein include, for example, cationic celluloses, cationic starches, and cationic guar gums.

### 6. Polyethylene Glycol

Polyethylene glycol can also be used as an additional component. The polyethylene glycols useful herein that are especially preferred are PEG-2M wherein n has an average value of about 2,000 (PEG-2M is also known as Polyox WSR^{®} N-10 from Union Carbide and as PEG-2,000); PEG-5M wherein n has an average value of about 5,000 (PEG-5M is also known as Polyox WSR^{®} N-35 and as Polyox WSR^{®} N-80, both from Union Carbide and as PEG-5,000 and Polyethylene Glycol 300,000); PEG-7M wherein n has an average value of about 7,000 (PEG-7M is also known as Polyox WSR^{®} N-750 from Union Carbide); PEG-9M wherein n has an average value of about 9,000 (PEG-9M is also known as Polyox WSR^{®} N-3333 from Union Carbide); and PEG-14M wherein n has an average value of about 14,000 (PEG-14M is also known as Polyox WSR^{®} N-3000 from Union Carbide). As used herein "n" refers to the number of ethylene oxide units in the polymer.

### METHOD OF USE

The hair conditioning compositions of the present invention may be used in conventional ways to provide conditioning and other benefits. Such method of use depends upon the type of composition employed but generally involves application of an effective amount of the product to the hair or scalp, which may then be rinsed from the hair or scalp (as in the case of hair rinses) or allowed to remain on the hair or scalp (as in the case of gels, lotions, creams, and sprays). "Effective amount" means an amount sufficient enough to provide a dry conditioning benefit. In general, from about 1g to about 50g is applied to the hair or scalp.

### PRODUCT FORMS

The hair conditioning compositions of the present invention can be in the form of rinse-off products or leave-on products, can be opaque, and can be formulated in a wide variety of product forms, including but not limited to creams, gels, emulsions, mousses and sprays.

The hair conditioning composition may optionally relate to aqueous emulsions comprising at least one polyorganosiloxane compound and/or at least one polyorganosiloxane composition as defined above. Such aqueous emulsions alternatively comprise at least 30 weight percent, alternatively at least 50 weight percent, still alternatively at least 80 weight percent water based on the total weight of the emulsions.

The hair conditioning compositions may be suitable for rinse-off products and leave-on products.

### NON-LIMITING EXAMPLES

The compositions illustrated in the following examples and tables exemplify specific embodiments of the compositions of the provided disclosure, but are not intended to be limiting thereof. Other modifications may be undertaken by the skilled artisan.

The compositions illustrated in the following examples are prepared by conventional formulation and mixing methods, an example of which is described below. All exemplified amounts are listed as weight percents and exclude minor materials such as diluents, preservatives, color solutions, imagery ingredients, botanicals, and so forth, unless otherwise specified.

Exemplary Silicone Quaternary Polymers A-E below all include the following structure and the substituents listed in Table 1:

M-Y-[-(N⁺R₂-T-N⁺R₂)-Y-]ₘ-[-(N⁺R²₂-A-E-A'-N⁺R²₂)-Y-]ₖ-M

**Table 1**

| **Variable** | | | | **Silicone Quaternary Polymer A** | **Silicone Quaternary Polymer B** | **Silicone Quaternary Polymer C** | **Silicone Quaternary Polymer D** | **Silicone Quaternary Polymer E** | | |
|---|---|---|---|---|---|---|---|---|---|---|
| **M** | | | | lauric ester | lauric ester | lauric ester | lauric ester | lauric ester | | |
| **Y** | | | | K-S-K | K-S-K | K-S-K | K-S-K | K-S-K | | |
| **K** | | | | CH₂-CHOH-CH₂-O-C₃H₆ | CH₂-CHOH-CH₂-O-C₃H₆ | CH₂-CHOH-CH₂-O-C₃H₆ | CH₂-CHOH-CH₂-O-C₃H₆ | CH₂-CHOH-CH₂-O-C₃H₆ | | |
| **S** | | | | PDMS block with 368 siloxane units | PDMS block with 368 siloxane units | PDMS block with 368 siloxane units | PDMS block with 450 siloxane units | PDMS block with 368 siloxane units | | |
| **R, R²** | | | | methyl | methyl | methyl | methyl | methyl | | |
| **T** | | | | C₆H₁₂ | C₆H₁₂ | C₆H₁₂ | C₆H₁₂ | C₆H₁₂ | | |
| **A** | | | | CH₂-COO- | CH₂-COO- | CH₂-COO- | CH₂-COO- | CH₂-COO- | | |
| **A'** | | CO-CH₂ | | CO-CH₂ | | CO-CH₂ | | CO-CH₂ | | CO-CH₂ |
| **E** | | Ethylene oxide (CH₂-CH₂-O) with average degree of ethoxylation of 2 | | Ethylene oxide (CH₂-CH₂-O) with average degree of ethoxylation of 34 | | Propylene oxide (CH₂-CH(CH₃)-O) with average degree of propoxylation of 3.5 | | Propylene oxide (CH₂-CH(CH₃)-O) with average degree of propoxylation of 3.5 | | Ethylene oxide (CH₂-CH₂-O) with average degree of ethoxylation of 2 |
| **Ratio of silicone blocks : alkylene oxide blocks** | | 1:1 | | 9:1 | | 9:1 | | 9:1 | 7:3 | |
| **Total Visccosity** | | 4700 mPa.s | | 2800 mPa.s | | 2600 mPa.s. | | 5400 mPa.s. | | 6000 mPa.s. |
| | | | | Silicone Emulsion | | | | | | |
| | | | | A | B | C | | D | | E |
| Water | | | | q.s. | q.s. | q.s. | | q.s. | | q.s. |
| C11-15 Pareth-5 ¹ | | | | 1.0 | 1.4 | 1.0 | | 2.0 | | 1.4 |
| C11-15 Pareth-12² | | | | 1.0 | 2.0 | | | | | 2.0 |
| Silicone A | | | | 20.0 | | | | | | |
| Silicone B | | | | | 10. 0 | | | | | |
| Silicone C | | | | | | 10.0 | | | | |
| Silicone D | | | | | | | | 20.0 | | |
| Silicone E | | | | | | | | | | 20.0 |
| Tergitol 15-S-5, from The Dow Chemical Company | | | | | | | | | | |
| ²Tergitol 15-S-12, from The Dow Chemical Company | | | | | | | | | | |
| | All ingredients in % as added | | | | | | | | | |
| Leave on Treatment Composition Examples | Leave on Treatment 1 | Leave on Treatment 2 | Leave on Treatment | 3 Leave on Treatment 4 | Leave on Treatment | 5 Leave on Treatment 6 | Leave on Treatment | 7 Leave on Treatment 8 | Leave on Treatment 9 | Leave on Treatment 10 |
| Ingredient | | | | | | | | | | |
| Water | QS | QS | QS | QS | QS | QS | QS | QS | QS | QS |
| Silicone Quaternary Polymer A | 1.0 | | | | | | | | | |
| Silicone Quaternary Polymer B | | 2.0 | | | | | | | | |
| Silicone Quaternary Polymer C | | | 0.5 | | | | | | | |
| Silicone Quaternary Polymer D | | | | 2.5 | | | | | | |
| Silicone Quaternary Polymer E | | | | | 0.75 | | | | | |
| Silicone Emulsion A | | | | | | 10.0 | | | | |
| Silicone Emulsion B | | | | | | | 10.0 | | | |
| Silicone Emulsion C | | | | | | | | 15.0 | | |
| Silicone Emulsion D | | | | | | | | | 20.0 | |
| Silicone Emulsion E | | | | | | | | | | 5.0 |
| SD Alcohol 40 ¹ | | 50.0000 | | | | 50.0000 | | | | 50.0000 |
| Polyacrylamide & C13- | | | 1.0000 | | | | 1.0000 | | | |
| 14 Isoparaffin & Laureth-7 ² | | | | | | | | | | |
| Dehydroxanthan Gum ³ | | 0.7500 | | | | 0.7500 | | | | 0.7500 |
| Sodium Polyacrylate Starch ⁴ | 0.5800 | | | 0.7000 | 0.5800 | | | 0.7000 | 0.5800 | |
| Glycerine ⁵ | 0.3000 | | | | 0.3000 | | | | 0.3000 | |
| Caffeine ⁶ | | 0.9375 | | | | 0.9375 | | | | 0.9375 |
| Niacinamide⁷ | | 3.1250 | | | | 3.1250 | | | | 3.1250 |
| D-Panthenol ⁸ | | 0.1875 | | | | 0.1875 | | | | 0.1875 |
| PEG-40 Hydrogenated Castor Oil ⁹ | | | | 0.3250 | | | | 0.3250 | | |
| Fragrance | 0.7000 | 0.3000 | 1.0000 | 0.1300 | 0.7000 | 0.3000 | 1.0000 | 0.1300 | 0.7000 | 0.3000 |
| Preservatives, pH adjusters | Up to 2% | Up to 2% | Up to 2% | Up to 2% | Up to 2% | Up to 2% | Up to 2% | Up to 2% | Up to 2% | Up to 2% |
| 1 | SD Alcohol 40B (200 Proof) supplier Equistar Chemicals | | | | | | | | | |
| 2 | Sepigel 305, 45% active, Supplier Seppic Inc. | | | | | | | | | |
| 3 | Amaze XT, 100% active, Supplier Akzo Nobel | | | | | | | | | |
| 4 | Makimousse 12, 100% active, Supplier KOBO Products | | | | | | | | | |
| 5 | Glycerine, 100% active, Supplier Spectrum Chemicals | | | | | | | | | |
| 6 | Caffeine USP, 100% active, Supplier BASF Pharmachemikalien Gmbh Kg | | | | | | | | | |
| 7 | Niacinamide USP FCC, 100% active, Supplier DSM Nutritional Products Inc | | | | | | | | | |
| 8 | D-Panthenol, 100% active, Supplier DSM Nutritional Products Inc | | | | | | | | | |
| 9 | Cremophor CO 40, 100% Active, Supplier BASF | | | | | | | | | |

Examples 1-2, 4-6 and 8-10 are not within the scope of the invention.

It is further noted that terms like "alternatively,""usually", "generally,""commonly,"and "typically"are not utilized herein to limit the scope of the claimed invention or to imply that certain features are critical, essential, or even important to the structure or function of the claimed invention. Rather, these terms are merely intended to highlight alternative or additional features that may or may not be utilized in a particular embodiment of the present invention.

For the purposes of describing and defining the present invention it is additionally noted that the term "sub-stantially" is utilized herein to represent the inherent degree of uncertainty that may be attributed to any quantitative comparison, value, measurement, or other representation. The term "substantially" is also utilized herein to represent the degree by which a quantitative representation may vary from a stated reference without resulting in a change in the basic function of the subject matter at issue.

Having described the invention in detail and by reference to specific embodiments thereof, it will be apparent that modifications and variations are possible. More specifically, although some aspects of the present invention are identified herein as preferred or particularly advantageous, it is contemplated that the present invention is not necessarily limited to these preferred aspects of the invention.

## Claims

1. A hair conditioning composition comprising:
a) a silicone polymer comprising:
i. one or more quaternary groups;
ii. at least one silicone block comprising greater than 200 siloxane units;
iii. at least one polyalkylene oxide structural unit; and
iv. at least one terminal ester group
wherein said silicone polymer has a viscosity from 500 to 50,000 mPa.s, measured by means of a glass capillary viscometer as set forth in Dow Corning Corporate Test Method CTM0004, and wherein said silicone polymer is defined by the following chemical structure:
M-Y-[-(N⁺R₂-T-N⁺R₂)-Y-]ₘ-[-(NR²-A-E-A'-NR²)-Y-]ₖ-M (Ia)
or
M-Y-[-(N⁺R₂-T-N⁺R₂)-Y-]ₘ-[-(N⁺R²₂-A-E-A'-N⁺R²₂)-Y-]ₖ-M (Ib)
wherein:
m is an average value of from above 0 to 100
k is an average value of from above 0 to 50
M represents a terminal group, comprising terminal ester groups selected from
-OC(O)-Z
-OS(O)₂-Z
-OS(O₂)O-Z
-OP(O)(O-Z)OH
-OP(O)(O-Z)₂
wherein Z is selected from monovalent organic residues having up to 40 carbon atoms,
wherein A and A' each are independently selected from a single bond or a divalent organic group having up to 10 carbon atoms and one or more hetero atoms, and
E is a polyalkylene oxide group of the general formula:
-[CH₂CH₂O]_{q}-[CH₂CH(CH₃)O]ᵣ-[CH₂CH(C₂H₅)O]ₛ-
with
q=0 to 200,
r=0 to 200,
s=0 to 200,
and q+r+s = 1 to 600,
R is selected from monovalent organic groups having up to 22 carbon atoms, and wherein the free valencies at the nitrogen atoms are bound to carbon atoms, R² is selected from hydrogen or R,
Y is a group of the formula:
-K-S-K- and -A-E-A'- or -A'-E-A-,
with
S= 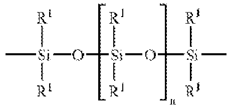
wherein R¹ = C₁-C₂₂-alkyl, C₁-C₂₂-fluoralkyl or aryl,
n=200 to 1000,
K is a bivalent or trivalent straight chain, cyclic and/or branched C₂-C₄₀ hydrocarbon residue, wherein T is selected from a divalent organic group having up to 20 carbon atoms and one or more hetero atoms; and
b) a polymeric thickener, wherein the polymeric thickener is a rheology modifier, wherein the rheology modifier is selected from the group consisting of acrylamide/ammonium acrylate copolymer (and) polyisobutene (and) polysorbate 20; acrylamide/sodium acryloyldimethyl taurate copolymer (and) isohexadecane (and) polysorbate 80; acrylates copolymer; acrylates/beheneth-25 methacrylate copolymer; acrylates/C10-C30 alkyl acrylate crosspolymer; acrylates/steareth-20 itaconate copolymer; ammonium polyacrylate (and) Isohexadecane (and) PEG-40 castor oil; C12-16 alkyl PEG-2 hydroxypropylhydroxyethyl ethylcellulose (HM-EHEC); carbomer; crosslinked polyvinylpyrrolidone (PVP); dibenzylidene sorbitol; hydroxyethyl ethylcellulose (EHEC); hydroxypropyl methylcellulose (HPMC); hydroxypropylcellulose (HPC); methylcellulose (MC); methylhydroxyethyl cellulose (MEHEC); PEG-150/decyl alcohol/SMDI copolymer; PEG-150/stearyl alcohol/SMDI copolymer; polyacrylamide (and) C13-14 isoparaffin (and) laureth-7; polyacrylate 13 (and) polyisobutene (and) polysorbate 20; polyacrylate crosspolymer-6; polyamide-3; polyquaternium-37 (and) hydrogenated polydecene (and) trideceth-6; polyurethane-39; sodium acrylate/acryloyldimethyltaurate/dimethylacrylamide crosspolymer (and) isohexadecane (and) polysorbate 60; and sodium polyacrylate.

2. The hair conditioning composition of claim 1, wherein said silicone block comprises from 300 to 500 siloxane units.

3. The hair conditioning composition of any preceding claim, wherein said silicone polymer is present in an amount of from 0.05% to 15% by weight of the composition.

4. The hair conditioning composition of any preceding claim, wherein said silicone polymer is present in an amount of from 0.1% to 10% by weight of the composition.

5. The hair conditioning composition of any preceding claim, wherein said silicone polymer is present in an amount of from 0.15% to 5% by weight of the composition.

6. The hair conditioning composition of any preceding claim whereby the K residues in the -K-S-K- moiety are identical or different, and are bound to the silicon atom of the residue S via a C-Si-bond.

7. The hair conditioning composition of any preceding claim wherein:
m is >0 to 10,
k is >0 to 10,
M is -OC(O)-Z,
Z is hydrocarbon chain with 0 to 40 carbons,
q = 0-50, r = 0-50, q + r is at least 1, s = 0,
R² is methyl, and
n = 300-500.

8. The hair conditioning composition of any preceding claim wherein the silicone polymer includes a viscosity from 500 to 5000 mPa.s.

9. A method of providing improved conditioning benefits to hair or skin, said method comprising the step of applying to said hair or skin the hair conditioning composition of any preceding claim.

## Patentansprüche

1. Haarkonditionierungszusammensetzung, umfassend:
a) ein Silikonpolymer, umfassend:
i. eine oder mehrere quartäre Gruppen;
ii. mindestens einen Silikonblock, umfassend mehr als 200 Siloxaneinheiten;
iii. mindestens eine Polyalkylenoxid-Struktureinheit; und
iv. mindestens eine endständige Estergruppe
wobei das Silikonpolymer eine Viskosität von 500 bis 50.000 mPa.s aufweist, gemessen mittels eines Glas-Kapillarviskosimeters, wie in dem Dow Corning Corporate-Prüfverfahren CTM0004 erläutert, und wobei das Silikonpolymer durch die folgende chemische Struktur definiert ist:
M-Y-[-(N⁺R₂-T-N⁺R₂)-Y-]ₘ-[-(NR²-A-E-A'-NR²)-Y-]ₖ-M (la)
oder
M-Y-[-(N⁺R₂-T-N⁺R₂)-Y-]ₘ-[-(N⁺R²₂-A-E-A'-N⁺R²₂)-Y-]ₖ-M (Ib)
wobei:
m ein Durchschnittswert von über 0 bis 100 ist
k ein Durchschnittswert von über 0 bis 50 ist
M eine endständige Gruppe darstellt, umfassend endständige Estergruppen, ausgewählt aus
-OC(O)-Z
-OS(O)₂-Z
-OS(O₂)O-Z
-OP(O)(O-Z)OH
-OP(O)(O-Z)₂
wobei Z ausgewählt ist aus einwertigen organischen Resten mit bis zu 40 Kohlenstoffatomen,
wobei A und A' jeweils unabhängig voneinander aus einer Einfachbindung oder einer zweiwertigen organischen Gruppe ausgewählt sind, die zu 10 Kohlenstoffatome und ein oder mehrere Heteroatomen aufweist; und
E eine Polyalkylenoxidgruppe der allgemeinen Formel ist:
-[CH₂CH₂O]_{q}-[CH₂CH(CH₃)O]ᵣ-[CH₂CH(C₂H₅)O]ₛ-
wobei
q = 0 bis 200,
r = 0 bis 200,
s = 0 bis 200,
und q+r+s = 1 bis 600,
R ausgewählt ist aus einwertigen organischen Gruppen, die bis zu 22 Kohlenstoffatome aufweisen und wobei die freien Valenzen an den Stickstoffatomen an Kohlenstoffatome gebunden sind, R² ausgewählt ist aus Wasserstoff oder R,
Y eine Gruppe der folgenden Formel ist:
-K-S-K- und -A-E-A'- oder -A'-E-A-,
wobei
S = 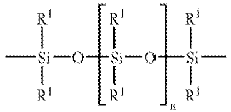
wobei R¹ = C₁-C₂₂-Alkyl, C₁-C₂₂-Fluoralkyl oder -Aryl,
n = 200 bis 1000,
K ein zweiwertiger oder dreiwertiger geradkettiger, cyclischer und/oder verzweigter C₂-C₄₀-Kohlenwasserstoffrest ist, wobei T ausgewählt ist aus einer zweiwertigen organischen Gruppe, die zu 20 Kohlenstoffatome und ein oder mehrere Heteroatome aufweist; und
b) ein polymeres Verdickungsmittel, wobei das polymere Verdickungsmittel ein Rheologiemodifikator ist, wobei der Rheologiemodifikator ausgewählt ist aus der Gruppe, bestehend aus Acrylamid/Ammoniumacrylat-Copolymer (und) Polyisobuten (und) Polysorbat 20; Acrylamid/Natriumacryloyldimethyltaurat-Copolymer (und) Isohexadecan (und) Polysorbat 80; Acrylatcopolymer; Acrylat/Beheneth-25-Methacrylatcopolymer; Acrylat/C10-C30-Alkylacrylat-Vernetzungspolymer; Acrylat/Steareth-20 Itaconatcopolymer; Ammoniumacrylat (und) Isohexadecan (und) PEG-40-Rizinusöl; C12-16-Alkyl-PEG-2-Hydroxypropylhydroxyethylethyl-Cellulose (HM-EHEC); Carbomer; vernetztes Polyvinylpyrrolidon (PVP); Dibenzylidensorbit; Hydroxyethylethylcellulose (EHEC); Hydroxypropylmethylcellulose (HPMC); Hydroxypropylcellulose (HPC); Methylcellulose (MC); Methylhydroxyethylcellulose (MEHC); PEG-150/Decylalkohol/SMDI-Copolymer; PEG-150/Stearylalkohol/SMDI-Copolymer; Polyacrylamid (und) C13-14-O-Isoparaffin (und) Laureth-7; Polyacrylat 13 (und) Polyisobuten (und) Polysorbat 20; Polyacrylatkreuzpolymer-6; Polyamid-3; Polyquaternium-37 (und) hydriertes Polydecen (und) Trideceth-6; Polyurethan-39; Natriumacrylat/Acryloyldimethyltaurat/Dimethylacrylamid-Kreuzpolymer (und) Isohexadecan (und) Polysorbat 60; und Natriumpolyacrylat.

2. Haarkonditionierungszusammensetzung nach Anspruch 1, wobei der Silikonblock von 300 bis 500 Siloxaneinheiten umfasst.

3. Haarkonditionierungszusammensetzung nach einem der vorstehenden Ansprüche, wobei das Silikonpolymer in einer Menge von 0,05 Gew.-% bis 15 Gew.-% der Zusammensetzung vorhanden ist.

4. Haarkonditionierungszusammensetzung nach einem der vorstehenden Ansprüche, wobei das Silikonpolymer in einer Menge von 0,1 Gew.-% bis 10 Gew.-% der Zusammensetzung vorhanden ist.

5. Haarkonditionierungszusammensetzung nach einem der vorstehenden Ansprüche, wobei das Silikonpolymer in einer Menge von 0,15 Gew.-% bis 5 Gew.-% der Zusammensetzung vorhanden ist.

6. Haarkonditionierungszusammensetzung nach einem der vorstehenden Ansprüche, wobei die K-Reste in der -K-S-K-Einheit gleich oder verschieden sind und über eine C-Si-Bindung an das Siliziumatom des Restes S gebunden sind.

7. Haarkonditionierungszusammensetzung nach einem der vorstehenden Ansprüche, wobei:
m >0 bis 10 ist,
k >0 bis 10 ist,
M -OC(O)-Z ist,
Z eine Kohlenwasserstoffkette mit 0 bis 40 Kohlenstoffatomen ist,
q = 0-50, r = 0-50, q + r mindestens 1 ist, s = 0,
R² Methyl ist, und
n = 300-500.

8. Haarkonditionierungszusammensetzung nach einem der vorstehenden Ansprüche, wobei das Silikonpolymer eine Viskosität von 500 bis 5000 mPa.s aufweist.

9. Verfahren zum Bereitstellen verbesserter Konditionierungsnutzwirkungen für Haar oder Haut, wobei das Verfahren den Schritt des Auftragens der Haarkonditionierungszusammensetzung nach einem der vorstehenden Ansprüche auf das Haar oder die Haut umfasst.

## Revendications

1. Composition de conditionnement des cheveux comprenant :
a) un polymère de silicone comprenant :
i. un ou plusieurs groupes quaternaires ;
ii. au moins un bloc de silicone comprenant plus de 200 motifs siloxane ;
iii. au moins un motif structural poly(oxyde d'alkylène) ; et
iv. au moins un groupe ester terminal
dans laquelle ledit polymère de silicone a une viscosité allant de 500 à 50 000 mPa.s, mesurée au moyen d'un viscosimètre à capillaire de verre tel que présenté dans le procédé de test Dow Corning Corporate CTM0004, et dans laquelle ledit polymère de silicone est défini par la structure chimique suivante :
M-Y-[-(N⁺R₂-T-N⁺R₂)-Y-]ₘ-[-(NR²-A-E-A'-NR²)-Y-]ₖ-M (la)
ou
M-Y-[-(N⁺R₂-T-N⁺R₂)-Y-]ₘ-[-(N⁺R²₂-A-E-A'-N⁺R²₂)-Y-]ₖ-M (Ib)
dans laquelle :
m est une valeur moyenne allant de plus de 0 à 100
k est une valeur moyenne allant de plus de 0 à 50
M représente un groupe terminal, comprenant des groupes ester terminaux choisis parmi
-OC(O)-Z
-OS(O)₂-Z
-OS(O₂)O-Z
-OP(O)(O-Z)OH
-OP(O)(O-Z)₂
dans laquelle Z est choisi parmi des résidus organiques monovalents ayant jusqu'à 40 atomes de carbone,
dans laquelle A et A' sont chacun indépendamment choisis parmi une liaison simple ou un groupe organique divalent ayant jusqu'à 10 atomes de carbone et un ou plusieurs hétéroatomes, et
E est un groupe poly(oxyde d'alkylène) de formule générale :
-[CH₂CH₂O]_{q}-[CH₂CH(CH₃)O]ᵣ-[CH₂CH(C₂H₅)O]ₛ-
avec
q=0 à 200,
r=0 à 200,
s=0 à 200,
et q+r+s = 1 à 600,
R est choisi parmi des groupes organiques monovalents ayant jusqu'à 22 atomes de carbone, et dans laquelle les valences libres au niveau des atomes d'azote sont liées à des atomes de carbone, R² est choisi parmi l'hydrogène ou R,
Y est un groupe de formule :
-K-S-K- et -A-E-A'-ou-A'-E-A-,
avec
S= 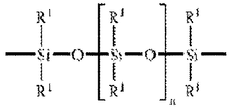
dans laquelle R¹ = alkyle en C₁ à C₂₂, fluoroalkyle en C₁ à C₂₂ ou aryle,
n=200 à 1000,
K est une chaîne linéaire bivalente ou trivalente, un résidu hydrocarboné cyclique et/ou ramifié en C₂ à C₄₀, dans laquelle T est choisi parmi un groupe organique divalent ayant jusqu'à 20 atomes de carbone et un ou plusieurs hétéroatomes ; et
b) un épaississant polymère, l'épaississant polymère étant un modificateur de rhéologie, le modificateur de rhéologie étant choisi dans le groupe constitué par le copolymère acrylamide/acrylate d'ammonium (et) le polyisobutène (et) polysorbate 20 ; acrylamide/copolymère d'acryloyldiméthyltaurate de sodium (et) isohexadécane (et) polysorbate 80 ; copolymère d'acrylates ; copolymère d'acrylates/méthacrylate de béhéneth-25 ; polymère réticulé d'acrylates/acrylate d'alkyle en C10 à C30 ; copolymère d'acrylates/itaconate de stéareth-20 ; polyacrylate d'ammonium (et) isohexadécane (et) PEG-40-huile de ricin ; C12 à 16-alkyl-PEG-2 hydroxypropylhydroxyéthyléthylcellulose (HM-EHEC) ; carbomère ; polyvinylpyrrolidone réticulée (PVP) ; dibenzylidènesorbitol ; hydroxyéthyléthylcellulose (EHEC) ; hydroxypropylméthylcellulose (HPMC) ; hydroxypropylcellulose (HPC) ; méthylcellulose (MC) ; méthylhydroxyéthylcellulose (MEHEC) ; copolymère de PEG-150/alcool décylique/SMDI ; copolymère de PEG-150/alcool stéarylique/SMDI ; polyacrylamide (et) isoparaffine C13 à 14 (et) laureth-7 ; polyacrylate 13 (et) polyisobutène (et) polysorbate 20 ; polymère réticulé de polyacrylate-6 ; polyamide-3 ; polyquaternium-37 (et) polydécène hydrogéné (et) tridéceth-6 ; polyuréthane-39 ; acrylate de sodium/acryloyldiméthyltaurate/diméthylacrylamide réticulé (et) isohexadécane (et) polysorbate 60 ; et polyacrylate de sodium.

2. Composition de conditionnement des cheveux selon la revendication 1, dans laquelle ledit bloc de silicone comprend de 300 à 500 motifs siloxane.

3. Composition de conditionnement des cheveux selon une quelconque revendication précédente, dans laquelle ledit polymère de silicone est présent en une quantité de 0,05 % à 15 % en poids de la composition.

4. Composition de conditionnement des cheveux selon une quelconque revendication précédente, dans laquelle ledit polymère de silicone est présent en une quantité de 0,1 % à 10 % en poids de la composition.

5. Composition de conditionnement des cheveux selon une quelconque revendication précédente, dans laquelle ledit polymère de silicone est présent en une quantité de 0,15 % à 5 % en poids de la composition.

6. Composition de conditionnement des cheveux selon une quelconque revendication précédente, selon laquelle les résidus K dans le fragment -K-S-K-sont identiques ou différents et sont liés à l'atome de silicium du résidu S par l'intermédiaire d'une liaison C-Si.

7. Composition de conditionnement des cheveux selon une quelconque revendication précédente, dans laquelle :
m va de >0 à 10,
k va de >0 à 10,
M est -OC(O)-Z,
Z est une chaîne hydrocarbonée avec 0 à 40 carbones,
q = 0-50, r = 0-50, q + r vaut au moins 1, s = 0,
R² est méthyle, et
n = 300-500.

8. Composition de conditionnement des cheveux selon une quelconque revendication précédente, dans laquelle le polymère de silicone inclut une viscosité allant de 500 à 5000 mPa.s.

9. Procédé de fourniture d'effets bénéfiques de conditionnement amélioré aux cheveux ou à la peau, ledit procédé comprenant l'étape d'application auxdits cheveux ou à ladite peau de la composition de conditionnement des cheveux selon une quelconque revendication précédente.
